# EUROPÄISCHE PATENTSCHRIFT

(11) **EP 0 751 118 B1**
(45) Veröffentlichungstag und Bekanntmachung des Hinweises auf die Patenterteilung: **13.10.1999**
(21) Anmeldenummer: 96110026.0
(22) Anmeldetag: 21.06.1996
(51) Int. Cl.: C07C 263/20, C07C 263/10

(54) **Verfahren zur Herstellung von Mischungen aus Diphenylmethandiisocyanaten und Polyphenyl-polymethylen-polyisocyanaten mit einer verminderten Iodfarbzahl und einem reduzierten Chlorgehalt**
Process for the preparation of mixtures of diphenylmethane diisocyanates and polyphenylmethylene polyisocyanates with a reduced iodic coloremetric measure and reduced chlorine content
Procédé de préparation de mélanges comprenant des diphénylméthanes-diisocyanates et des polyphénylméthylène-polyisocyanates ayant une mesure colorémétrique à base d'iode réduite et à basse teneur en chlore

(30) Priorität: 30.06.1995 DE 19523851
(43) Veröffentlichungstag der Anmeldung: 02.01.1997
(73) Patentinhaber: BASF Aktiengesellschaft, 67063 Ludwigshafen (DE)
(72) Erfinder: Scherzer, Dietrich, Dr., 67433 Neustadt (DE); Bruchmann, Bernd, Dr., 67069 Ludwigshafen (DE); Minges, Roland, Dr., 67269 Grünstadt (DE); Keller, Peter, Dr., 69493 Hirschberg (DE); Van Pee, Willy, Dr., 2950 Kapellen (BE); Heider, Wolfgang, 67434 Neustadt (DE); Pohl, Siegmund, Dr., 67069 Ludwigshafen (DE); Otto, Bernhard, Dr., 67117 Limburgerhof (DE); Jacobs, Paul, Dr., 2970 s'Gravenwezel (BE); Seyfert, Wilfried, Dr., 67273 Weisenheim (DE)

(56) Entgegenhaltungen:
- EP-A- 0 445 602
- EP-A- 0 467 125

## Beschreibung

Erfindungsgegenstand ist ein Verfahren zur Herstellung von Mischungen aus Diphenylmethan-diisocyanaten und Polyphenylpolymethylen-polyisocyanaten, sogenanntes Roh-MDI, mit einer reduzierten Iodfarbzahl und einem verminderten Chlorgehalt durch Umsetzung der entsprechenden Mischungen aus Diphenylmethan-diaminen und Polyphenyl-polymethylen-polyaminen, sogenanntes Roh-MDA, mit Phosgen in Gegenwart mindestens eines inerten organischen Lösungsmittels, wobei man der Reaktionsmischung nach beendeter Phosgenierung eine Mischung aus Wasser und mindestens einem ein- oder mehrwertigen Polyoxyalkylenalkohol in einer wirksamen Menge einverleibt.

Roh-MDI, einer der technisch bedeutendsten Ausgangsstoffe zur Herstellung von Polyisocyanat-polyadditionsprodukten, beispielsweise Urethan- oder Urethan- und Isocyanuratgruppen enthaltenden Schaumstoffen, und von 4,4'-Diphenylmethan-diisocyanat, einer wichtigen Aufbaukomponente zur Herstellung von Polyurethan(PU)-Elastomeren, -Fasern, -Dichtungsmassen, -Klebstoffen u.a. wird bekanntermaßen hergestellt durch Phosgenierung von Roh-MDA, üblicherweise in Gegenwart eines inerten organischen Lösungsmittels. Roh-MDA wird seinerseits erhalten durch Kondensation von Anilin und Formaldehyd in Gegenwart von sauren Katalysatoren, wobei in Abhängigkeit von den gewählten Mengenverhältnissen der Ausgangsstoffe und den Reaktionsbedingungen sowie den unterschiedlichen Verfahren der prozentuale Anteil an Diphenylmethan-diaminen und den homologen Polyphenyl-polymethylen-polyaminen sowie ihren Isomeren gesteuert werden kann (Kunststoff-Handbuch, Band 7, Polyurethane, 1. Auflage 1966 und 2. Auflage 1983, Carl-Hanser-Verlag, München, Wien). Wird die Kondensation von Anilin und Formaldehyd z.B. in Gegenwart von schwach sauren Katalysatoren durchgeführt, so erhält man Roh-MDA-Gemische mit einem relativ hohen Anteil an 2,2'- und 2,4'-Diamino-diphenylmethanen, während Roh-MDA-Gemische mit einem großen Gehalt an 4,4'-Diamino-diphenylmethan und gleichzeitig geringem Anteil an 2,4'-Diamino-diphenylmethan nur in Gegenwart von größeren Mengen stark saurer Katalysatoren, vorzugsweise von starken Mineralsäuren, wie z.B. Salzsäure, hergestellt werden können.

Das Verhältnis von Diamino-diphenylmethan-Isomeren zu den höheren Homologen im Roh-MDA ist ferner abhängig vom Anilin-Formaldehyd-Verhältnis und der Kondensationstemperatur, wobei größere Anilin-Formaldehyd-Verhältnisse und niedrige Kondensationstemperaturen hohe Diamino-diphenylmethangehalte ergeben (CA-A-770 026).

Nachteilig an diesen Herstellungsverfahren, die in einer Vielzahl von Literatur- und Patentpublikationen beschrieben werden, ist die Bildung von mehr oder weniger stark gefärbten Roh-MDA, deren Farbe von schwarz über dunklere und hellere Brauntöne bis zu ocker variieren kann. Nachteilig ist ferner, daß diese Verfärbungen auch durch die anschließende Phosgenierung zur Herstellung der entsprechenden Roh-MDI nicht oder nur unzureichend vermindert werden und das gebildete Roh-MDI nicht durch Destillation gereinigt werden kann. Diese unerwünschte Verfärbung wird außerdem in den Folgeprodukten wirksam, so daß auch die aus gefärbtem Roh-MDI hergestellten gegebenenfalls zellhaltigen Polyisocyanat-Polyadditionsprodukte nicht farblos sind. Obgleich die Eigenfarbe der Polyisocyanat-Polyadditionsprodukte deren mechanische Eigenschaften nicht negativ beeinflußt, werden vom Verbraucher im wesentlichen farblose Produkte gewünscht.

Es hat daher nicht an Versuchen gefehlt, die Verfärbungen von Roh-MDI zu vermindern und die hergestellten Polyisocyanate durch geeignete Verfahrensmaßnahmen oder Zusatzstoffe zu stabilisieren.

Nach Angaben der US-A-2 885 420 können organische Polyisocyanate gegen eine Verfärbung durch die Zugabe von 0,01 bis 0,5 Gew.-%, bezogen auf das Polyisocyanatgewicht, eines aromatischen, cycloaliphatischen oder aliphatischen Ethers oder Thioethers stabilisiert werden.

Zur Beseitigung von als Gelbildungskatalysatoren wirkenden Verunreinigungen in organischen Diisocyanatlösungen werden diesen gemäß DE-A-1 280 855 (GB 1 097 219) etwa 0,001 bis 1 Gew.-%, bezogen auf das Gewicht des Diisocyanats, Phosphorsäure zugesetzt.

Die GB-B-1 465 014 beschreibt den Zusatz von Glycidol in einer Menge von 0,001 bis 0,25 Gew.-%, bezogen auf das Diisocyanatgewicht, zur Verbesserung der Lagerstabilität von destillierten Diphenylmethan-diisocyanaten.

Die EP-B-0 183 976 (US-A-4 677 221) betrifft ein Verfahren zur Herstellung von wärmefarbbeständigen (cyclo)aliphatischen Diisocyanaten, wobei man technisches Diisocyanat mit aliphatisch und/ oder cycloaliphatisch gebundenen Isocyanatgruppen in Gegenwart von 0,1 bis 3 Gew.-% einer in dem Diisocyanat löslichen Verbindung, welche mindestens 3 Gew.-% an Struktureinheiten der Formel -NH-CO- aufweist, während eines Zeitraums von bis zu 5 Stunden auf eine Temperatur von 100 bis 220°C erhitzt und anschließend das so behandelte Diisocyanat durch Destillation reinigt. Das Verfahren ist nicht auf die Behandlung von Roh-MDI übertragbar, da, wie bereits ausgeführt wurde, dieses nicht destillierbar ist.

Nach Angabe der US-A-4 465 639 werden Roh-MDI nach beendeter Phosgenierung, aber vor der vollständigen Abtrennung des Phosgens, 0,1 bis 5 Gew.-% Wasser, bezogen auf das Polyisocyanatgewicht der Reaktionsmischung, einverleibt. Durch diese Maßnahme kann die Farbe des Roh-MDI und der daraus hergestellten PU-Schaumstoffe aufgehellt werden. Ferner wird der Anteil an höhermolekularen MDI-Homologen im Roh-MDI beträchtlich erniedrigt und ihre Viskosität reduziert. Obgleich auf diese Weise die Iodfarbzahl des Roh-MDI gesenkt werden kann, sind mit dieser Methode auch erhebliche Nachteile verbunden. Durch die Gegenwart von Wasser wird die korrodierende Wirkung der Chlor, Chlorwasserstoff und Phosgen enthaltenden Reaktionsmischung auf die Apparate der Produktionsanlage beträchtlich verstärkt und dadurch das Leckagerisiko, verbunden mit einem Ausbruch von toxischem Phosgen oder einer phosgenhaltigen Reaktionsmischung, erhöht. Aus Sicherheitsgründen wird daher nach vorherrschender Lehrmeinung Feuchtigkeit in jeder Form bei der Phosgenierung zweckmäßigerweise im wesentlichen vollständig ausgeschlossen.

Anstelle von Wasser können gemäß EP-A-0 467 125 zur Verminderung der Iodfarbzahl von Roh-MDI der Reaktionsmischung nach beendeter Phosgenierung ein- oder mehrwertige Polyoxyalkylenalkohole oder Mischungen davon in einer wirksamen Menge einverleibt werden. Obgleich nach diesem Verfahren die Iodfarbzahl des Roh-MDI beträchtlich reduziert werden kann, z.B. auf Werte von kleiner als 60, vorzugsweise von 35 bis 20, weist auch diese Methode Nachteile auf. Nachteilig ist beispielsweise, daß durch den Zusatz der Polyoxyalkylenalkohole der Isocyanatgehalt des Roh-MDI abnimmt und der Chlorgehalt, insbesondere der Gesamtchlorgehalt ansteigt und sich die Viskosität mit zunehmender Lagerzeit erhöhen kann.

Die Aufgabe der vorliegenden Erfindung bestand darin, die vorgenannten Nachteile auf ein Mindestmaß zu reduzieren und lagerbeständige Roh-MDI mit einem möglichst großen Isocyanatgehalt, einem möglichst geringen Gesamtchlorgehalt und einer möglichst niedrigen Iodfarbzahl herzustellen.

Diese Aufgabe konnte überraschenderweise gelöst werden durch den Zusatz einer Mischung aus Wasser und mindestens einem ein- und/ oder mehrwertigen Polyoxyalkylenalkohol zu der phosgenhaltigen oder zweckmäßigerweise überwiegend phosgenfreien Roh-MDI enthaltenden Reaktionsmischung nach beendeter Phosgenierung.

Gegenstand der Erfindung ist somit ein Verfahren zur Herstellung von Roh-MDI mit einer reduzierten Iodfarbzahl und einem verminderten Chlorgehalt durch Umsetzung der entsprechenden Roh-MDA mit Phosgen in Gegenwart mindestens eines inerten organischen Lösungsmittels bei erhöhter Temperatur, nach beendeter Phosgenierung Abtrennung des überschüssigen Phosgens und Lösungsmittels und thermischer Behandlung des erhaltenen Reaktionsprodukts, das dadurch gekennzeichnet ist, daß man der Reaktionsmischung nach beendeter Phosgenierung in Gegenwart oder Abwesenheit des Phosgens eine Mischung, die enthält oder vorzugsweise besteht aus mehr als 0,01 bis 3,0 Gew.-% Wasser und 0,05 bis 5 Gew.-%, jeweils bezogen auf das Gewicht der Mischung aus Diphenylmethan-diisocyanaten und Polyphenyl-polymethylen-polyisocyanaten in der Reaktionsmischung, mindestens eines ein- oder mehrwertigen Polyoxyalkylenalkohols oder Gemischen davon, einverleibt.

Nach dem erfindungsgemäßen Verfahren können überraschenderweise Roh-MDI hergestellt werden mit einer Iodfarbzahl, die niedriger ist als bei der alleinigen Verwendung von Wasser als Zusatzstoff und ungefähr dem Iodfarbzahlniveau entspricht, das mit der alleinigen Verwendung von Polyoxyalkylenalkoholen oder deren Gemischen erzielt werden kann. Überraschend war ferner, daß durch den erfindungsgemäßen Zusatz einer Mischung aus Wasser und mindestens einem Polyoxyalkylenalkohol im Vergleich zur alleinigen Verwendung mindestens eines Polyoxyalkylenalkohols im Roh-MDI der Isocyanatgehalt erhöht wird und der Gehalt an leicht hydrolysierbarem und schwer hydrolysierbarem Chlor sowie insbesondere der Gesamtchlorgehalt vermindert wird. Dieser synergistische Effekt war ebensowenig vorhersehbar wie die Unterbindung der korrodierenden Wirkung der Chlor, Chlorwasserstoff und Phosgen enthaltenden Reaktionsmischung auf die Apparate aus Stahl der Produktionsanlage in Gegenwart der zugesetzten erfindungsgemäß erforderlichen Mengen an Wasser und Polyoxyalkylenalkoholen.

Die nach dem erfindungsgemäßen Verfahren hergestellten Mischungen aus Diphenylmethan-diisocyanaten (MDI) und Polyphenyl-polymethylen-polyisocyanaten besitzen vorteilhafterweise einen MDI-Isomerengehalt von 30 bis 90 Gew.-%, vorzugsweise von 30 bis 70 Gew.-%, einem Isocyanatgehalt von 30 bis 33 Gew.-%, vorzugsweise von 30,5 bis 32,5 Gew.-%, jeweils bezogen auf das Roh-MDI-Gewicht, eine Iodfarbzahl von 20 bis 60, vorzugsweise von 25 bis 40, einen Gehalt an leicht hydrolysierbarem Chlor von 100 bis 300 ppm, vorzugsweise von 120 bis 200 ppm, an schwer hydrolysierbarem Chlor von 500 bis 2000 ppm, vorzugsweise von 800 bis 1400 ppm einen Gesamtchlorgehalt von 1500 bis 4000 ppm, vorzugsweise von 1700 bis 3000 ppm, und eine Viskosität von maximal 2000 mPa·s, vorzugsweise von 40 bis 350 mPa·s, gemessen bei 25°C nach DIN 53 019.

Roh-MDI mit solchen Isomeren- und Homologenzusammensetzungen können, wie bereits ausgeführt wurde, durch Phosgenierung von Roh-MDA mit entsprechenden Produktzusammensetzungen in Gegenwart mindestens eines inerten organischen Lösungsmittels nach bekannten Verfahren hergestellt werden.

Geeignete Roh-MDA werden vorteilhafterweise erhalten durch Kondensation von Anilin und Formaldehyd in einem Molverhältnis von 6 bis 1,6 : 1, vorzugsweise von 3 bis 1,9 : 1 und einem Molverhältnis von Anilin zu sauren Katalysatoren von 1 : 0,98 bis 0,01, vorzugsweise 1 : 0,8 bis 0,2.

Der Formaldehyd wird vorzugsweise in Form einer wäßrigen Lösung, z.B. als handelsübliche 30 bis 50 gew.-%ige Lösung, verwendet.

Als saure Katalysatoren haben sich Protonendonatoren, wie z.B. saure Ionenaustauscherharze oder starke organische und vorzugsweise anorganische Säuren bewährt. Als starke Säuren sind hierbei solche mit einem pKs-Wert kleiner als 1,5 - bei mehrbasischen Säuren gilt dieser Wert für die erste Wasserstoffdissoziation - zu verstehen. Beispielhaft genannt seien Salzsäure, Schwefelsäure, Phosphorsäure, Fluorsulfonsäure und Oxalsäure. Chlorwasserstoff kann auch gasförmig eingesetzt werden. Vorzugsweise zur Anwendung kommt wäßrige Salzsäure in Konzentrationen von etwa 25 bis 31 Gew.-%.

In Betracht kommende Verfahren zur Roh-MDA-Herstellung werden beispielsweise beschrieben in CA-A-700 026, DE-B-22 27 110 (US-A-4 025 557), DE-B-22 38 920 (US-A-3 996 283), DE-B-24 26 116 (GB-A-1 450 632), DE-A-12 42 623 (USA-A-3 478 099), GB-A-1 064 559 und DE-A-32 25 125.

Als andere Ausgangskomponente zur Herstellung von Roh-MDI wird Phosgen verwendet. Das gasförmige Phosgen kann als solches oder in Verdünnung mit unter den Reaktionsbedingungen inerten Gasen, wie Stickstoff, Kohlenmonoxid u.a. eingesetzt werden. Das Molverhältnis von Roh-MDA zu Phosgen wird zweckmäßigerweise so bemessen, daß pro NH₂-Gruppe 1 bis 10 Mol, vorzugsweise 1,3 bis 4 Mol, Phosgen in der Reaktionsmischung vorliegen.

Als inerte organische Lösungsmittel kommen Verbindungen in Betracht, in welchen das Roh-MDA und das Phosgen mindestens teilweise löslich sind.

Als Lösungsmittel vorzüglich bewährt haben sich chlorierte, aromatische Kohlenwasserstoffe, beispielsweise Monochlorbenzol, Dichlorbenzole wie z.B. o-Dichlorbenzol, p-Dichlorbenzol, Trichlorbenzole, die entsprechenden Toluole und Xylole, Chlorethylbenzol, Monochlordiphenyl, α- bzw. β-Naphthylchlorid und Phthalsäuredialkylester, wie iso-Diethylphthalat. Insbesondere Anwendung finden als inerte Lösungsmittel Monochlorbenzol, Dichlorbenzole oder Mischungen dieser Chlorbenzole. Die Lösungsmittel können einzeln oder als Gemische verwendet werden. Zweckmäßigerweise wird ein Lösungsmittel verwendet, das einen niedrigeren Siedepunkt besitzt als die MDI-Isomeren, damit das Lösungsmittel leicht durch Destillation vom Roh-MDI abgetrennt werden kann. Die Menge an Lösungsmittel wird zweckmäßig so bemessen, daß die Reaktionsmischung einen Roh-MDI-Gehalt von 2 bis 40 Gew.-%, vorzugsweise zwischen 5 und 20 Gew.-%, bezogen auf das Gesamtgewicht der Reaktionsmischung, aufweist.

Das Roh-MDA kann als solche oder gelöst in organischen Lösungsmitteln zur Anwendung kommen. Insbesondere verwendet man jedoch Roh-MDA-Lösungen mit einem Roh-MDA-Gehalt von 2 bis 40 Gew.-%, vorzugsweise von 5 bis 20 Gew.-%, bezogen auf das Gesamtgewicht der Aminlösung.

Zur Verminderung der Iodfarbzahl werden der phosgenhaltigen Reaktionsmischung erfindungsgemäß Mischungen aus Wasser und mindestens einem ein- oder mehrwertigen Polyoxyalkylenalkohol oder Gemischen aus einwertigen, mehrwertigen oder ein- und mehrwertigen Polyoxyalkylenalkoholen in einer wirksamen Menge einverleibt.

Als Wasser kann hierfür beispielsweise Trinkwasser verwendet werden. Zweckmäßigerweise verwendet wird jedoch metallionenfreies Wasser, wie z.B. destilliertes Wasser oder mittels eines Ionenaustauschers gereinigtes Wasser.

Geeignete einwertige und mehrwertige Polyoxyalkylenalkohole besitzen zweckmäßigerweise eine Hydroxylzahl von 20 bis 1800, vorzugsweise von 100 bis 1100 und insbesondere von 200 bis 800 und die mehrwertigen Polyoxyalkylenalkohole weisen vorteilhafterweise eine Funktionalität vorzugsweise von 2 bis 8 und insbesondere von 2 bis 3 auf. Vorzüglich bewährt haben sich Polyoxyalkylenalkohole, die in den inerten organischen Lösungsmitteln zur Herstellung des Roh-MDI's, vorzugsweise Monochlorbenzol, Dichlorbenzole oder Mischungen davon, in den erforderlichen wirksamen Mengen zumindest teilweise, vorzugsweise jedoch vollständig löslich sind.

Die Polyoxyalkylenalkohole können nach bekannten Verfahren, beispielsweise durch anionische Polymerisation mit Alkalihydroxiden, wie z.B. Natrium- oder Kaliumhydroxid oder Alkalialkoholaten, wie Natriummethylat, Natrium- oder Kaliumethylat oder Kaliumisopropylat als Katalysatoren und unter Zusatz mindestens eines Startermoleküls, das mindestens ein, vorzugsweise 2 bis 8 und inbesondere 2 oder 3 reaktive Wasserstoffatome gebunden enthält, oder durch kationische Polymerisation mit Lewis-Säuren, wie Antimonpentachlorid, Borfluorid-Etherat u.a. oder Bleicherde als Katalysatoren aus einem oder mehreren Alkylenoxiden mit 2 bis 4 Kohlenstoffatomen im Alkylenrest hergestellt werden.

Geeignete Alkylenoxide sind beispielsweise Tetrahydrofuran, 1,3-Propylenoxid, 1,2- bzw. 2,3-Butylenoxid, Styroloxid und vorzugsweise Ethylenoxid und 1,2-Propylenoxid. Die Alkylenoxide können einzeln, alternierend nacheinander oder als Mischungen verwendet werden. Als Startermoleküle kommen beispielsweise in Betracht: Alkanole mit verzweigtkettigen oder vorzugsweise linearen Alkylresten mit 1 bis 10, vorzugsweise 1 bis 4 Kohlenstoffatomen, wie z.B. Methanol, Ethanol, n- und iso-Propanol, n- und sek.-Butanol, Pentanol, Hexanol, n- und iso-Octanole, mehrwertige, vorzugsweise zwei- bis achtwertige, insbesondere zwei- und/oder dreiwertige Alkohole oder Alkylenglykole wie z.B. Ethandiol, Propandiol-1,2 und -1,3, Butandiol-1,4, Pentandiol-1,5, Hexandiol-16, Glycerin, Trimethylol-propan, Pentaerythrit, Sorbit und Saccharose sowie Diethylenglykol, Dipropylenglykol und Wasser.

Als ein- oder mehrwertige Polyoxyalkylenalkohole kommen beispielsweise in Betracht: Polyoxytetramethylen-, Polyoxytetramethylen-polyoxypropylen-, Polyoxytetramethylen-polyoxyethylen-, Polyoxypropylen-, Polyoxypropylen-polyoxyethylen- und Polyoxyethylenalkohole. Besonders bewährt haben sich jedoch und daher vorzugsweise Verwendung finden mehrwertige Polyoxyalkylenalkohole mit einer Funktionalität von 2 bis 3 und einer Hydroxylzahl von 200 bis 800, insbesondere von 240 bis 600, zweckmäßigerweise solche, hergestellt aus Ethylenoxid, 1,2-Propylenoxid oder 1,2-Propylenoxid und Ethylenoxid, wobei die erhaltenen Polyoxypropylen-polyoxyethylenalkohole die Ethylenoxid- und 1,2-Propylenoxideinheiten in statistischer Verteilung oder blockweise oder in statistischer Verteilung mit Ethylenoxidendblöcken gebunden enthalten können. Die ein- oder mehrwertigen Polyoxyalkylenalkohole können einzeln oder in Form von Mischungen verwendet werden.

Geeignet sind selbstverständlich auch Mischungen aus ein- und mehrwertigen Polyoxyalkylenalkoholen.

Erfindungsgemäß verwendet werden mehr als 0,01 bis 0,3 Gew.-%, vorzugsweise mehr als 0,01 Gew.-% bis weniger als 0,1 Gew.-% Wasser, bezogen auf das Gewicht des Roh-MDI in der Reaktionsmischung und von 0,05 bis 5 Gew.-%, bezogen auf das Gewicht des Roh-MDI in der Reaktionsmischung, mindestens eines Polyoxyalkylenalkohols, wobei Polyoxyalkylenalkohole mit einer Hydroxylzahl von kleiner als 380 zweckmäßigerweise in einer Menge von 0,1 bis 5 Gew.-%, insbesondere 0,2 bis 3 Gew.-%, solche mit einer Hydroxylzahl von 200 bis 800 zweckmäßigerweise in einer Menge von 0,1 bis 3 Gew.-%, insbesondere 0,2 bis 1,5 Gew.-% und Polyoxyalkylenalkohole mit einer Hydroxylzahl von größer als 800 zweckmäßigerweise in einer Menge von 0,05 bis 1,5 Gew.-%, insbesondere 0,1 bis 1,0 Gew.-%, jeweils bezogen auf das Gewicht des lösungsmittelfreien Roh-MDI, eingesetzt werden.

Die Polyoxyalkylenalkohole können in reiner oder handelsüblicher Qualität eingesetzt werden. Geeignet sind jedoch auch Polyoxyalkylenalkohole mit höheren als den handelsüblichen Wassergehalten, sofern der Wassergehalt in der zugesetzten Polyoxyalkylenalkoholmenge nicht die erfindungsgemäß erforderliche Wassermenge übersteigt. Bei Verwendung derartiger Polyoxyalkylenalkohole mit einem erhöhten Wassergehalt kann in Abhängigkeit vom Wassergehalt im Polyoxyalkylenalkohol gegebenenfalls auf eine getrennte zusätzliche Wasserzugabe verzichtet werden.

Nach Abtrennung des Phosgens und des inerten Lösungsmittels können dem Wasser und ein- und/oder mehrwertige Polyoxyalkylenalkohole und/oder Reaktionsprodukte, erhältlich aus Wasser und diesen ein- und/oder mehrwertigen Polyoxyalkylenalkoholen und Roh-MDI, enthaltenden Roh-MDI gegebenenfalls noch mindestens ein Antioxidans auf Phenolbasis, mindestens ein Arylphosphit oder eine Mischung dieser Stabilisatoren hinzugefügt werden. Sofern diese Stabilisatoren, die in Verbindung mit den erfindungsgemäß verwendeten Mischungen aus Wasser und Polyoxyalkylenalkoholen eine zusätzliche Reduzierung der Iodfarbzahl bewirken können, Anwendung finden, werden sie zweckmäßigerweise in einer Menge von 0 bis maximal 5 Gew.-%, vorzugsweise von 0,01 bis 3 Gew.-% und insbesondere 0,1 bis 1,0 Gew.-%, bezogen auf das Gewicht des Roh-MDI, eingesetzt.

Als geeignete Antioxidantien auf Phenolbasis kommen beispielsweise in Betracht: Styrolisierte Phenole, das sind Phenole, die in 2- oder 4-Stellung oder in 2- und 4- und/oder 6-Stellung eine 1-Phenyl-ethylgruppe gebunden enthalten, Bis[2-hydroxy-5-methyl-3-tert.-butylphenyl] methan, 2,2-Bis[4-hydroxy-phenyl]-propan, 4,4'-Dihydroxy-biphenyl, 3,3'-Dialkyl- bzw. 3,3',5,5'-Tetraalkyl-4,4'-dihydroxy-biphenyl, Bis-[4-hydroxy-2-methyl-5-tert.-butylphenyl]-sulfid, Hydrochinon, 4-Methoxy-, 4-tert.-Butoxy-oder 4-Benzyloxy-phenol, Gemische aus 4-Methoxy-2- bzw. -3-tert.-butylphenol, 2,5-Dihydroxy-1-tert.-butyl-benzol, 2,5-Dihydroxy-1,4-di-tert.-butylbenzol, 4-Methoxy-2,6-di-tert.-butylphenol und vorzugsweise 2,6-Di-tert.-butyl-p-kresol.

Als Arylphosphite bewährt haben sich Tri-(alkylphenyl)-phosphite mit 1 bis 10 C-Atomen im Alkylrest, wie z.B. Tri-(methylphenyl)-, Tri-(ethylphenyl)-, Tri-(n-propylphenyl)-, Tri-(isopropylphenyl)-, Tri-(n-butylphenyl)-, Tri-(sek.-butylphenyl)-, Tri-(tert.-butylphenyl)-, Tri-(pentylphenyl)-, Tri-(hexylphenyl)-, Tri-(2-ethyl-hexylphenyl)-, Tri-(octylphenyl)-, Tri-(2-ethyl-octylphenyl)-, Tri-(decylphenyl)-phosphit und vorzugsweise Tri-(nonylphenyl)-phosphit, und insbesondere Triphenylphosphit.

Zur Herstellung der Roh-MDI mit reduzierter Iodfarbzahl und vermindertem Chlorgehalt nach dem erfindungsgemäßen Verfahren werden die entsprechenden Roh-MDA zweckmäßigerweise bei einer Temperatur im Bereich von 90 bis 220°C, vorzugsweise von 120 bis 180°C, bei-Normaldruck oder vorzugsweise unter erhöhtem Druck, z.B. bei 1 bis 10 bar, vorzugsweise 2 bis 5 bar, phosgeniert. Die bei dem erfindungsgemäßen Verfahren angewandte Temperatur liegt über der Zersetzungstemperatur der als Zwischenprodukte durch die Reaktion von Roh-MDA mit Phosgen gebildeten Carbamidsäurechloride. Einer Erhöhung des Drucks sind nur technische und gegebenenfalls sicherheitstechnische Grenzen gesetzt, wobei jedoch mit einer größeren Druckerhöhung keine Ausbeutesteigerungen mehr verbunden sind.

Nach beendeter Phosgenierung kann der Reaktionsmischung, die mindestens ein inertes organisches Lösungsmittel, gelöstes Roh-MDI, überschüssiges Phosgen, Chlorwasserstoff sowie Nebenprodukten der Phosgenierung enthält, bei einer Temperatur beispielsweise von 20 bis 220°C, vorzugsweise von 90 bis 200°C und insbesondere 125 bis 165°C die Mischung aus Wasser und mindestens einem ein- und/oder vorzugsweise mehrwertigen Polyoxyalkylenalkohol, insbesondere eine Mischung aus Wasser und Polyoxypropylen-polyol oder wasserhaltige Polyoxyalkylenalkohole oder wasserhaltige Polyoxyalkylenalkoholgemische hinzugefügt werden. Nach einer anderen Verfahrensvariante, die vorzugsweise Anwendung findet, wird nach beendeter Phosgenierung aus der Reaktionsmischung zunächst das überschüssige Phosgen nahezu vollständig abgetrennt. Danach wird der Reaktionsmischung, die im wesentlichen aus mindestens einem inerten organischen Lösungsmittel, gelösten Roh-MDI, nicht abgetrennten Phosgenresten, Chlorwasserstoff sowie Nebenprodukten der Phosgenierung besteht, die erfindungsgemäß geeignete Mischung aus Wasser und mindestens einem Polyoxyalkylenalkohol in einer wirksamen Menge bei einer Temperatur im vorgenannten Bereich einverleibt. Nach einer Verweilzeit z.B. von 0,1 bis 45 Minuten, vorzugsweise von 2 bis 25 Minuten bei einer Temperatur von 20 bis 180°C, vorzugsweise von 70 bis 180°C können das überschüssige Phosgen oder, nach der bevorzugten Verfahrensweise, die noch vorliegenden Phosgenreste bei Normaldruck und anschließend bei einer Temperatur von 30 bis 180°C, vorzugsweise von 50 bis 180°C das inerte organische Lösungsmittel oder Mischungen davon unter vermindertem Druck, z.B. bei einem Druck von 0,01 bis 100 mbar, vorzugsweise von 0,1 bis 50 mbar im wesentlichen vollständig, vorzugsweise durch Destillation, abgetrennt werden.

Den Wasser und ein- und/oder vorzugsweise mehrwertige Polyoxyalkylenalkohole und/oder insbesondere Reaktionsprodukten aus Roh-MDI mit Wasser und diesen Polyoxyalkylenalkoholen enthaltenden Roh-MDI's können nunmehr, sofern dies zweckdienlich erscheint, mindestens ein Antioxidans auf Phenolbasis und/oder mindestens ein Arylphosphit in einer wirksamen Menge hinzugefügt werden. Danach werden die auf diese Weise behandelten Roh-MDI zur Entchlorierung auf eine Temperatur z.B. von 100 bis 250°C, vorzugsweise von 140 bis 200°C erhitzt und bei dieser Temperatur unter einem Druck z.B. von 0,01 bis 100 mbar, vorzugsweise von 0,1 bis 20 mbar mindestens 5 Minuten und insbesondere 5 bis 45 Minuten, behandelt. Nach der Abkühlung auf 60°C wird das Roh-MDI der Zwischenlagerung zugeführt und dort weiter abkühlen gelassen.

Die nach dem erfindungsgemäßen Verfahren hergestellten Roh-MDI besitzen eine deutlich reduzierte Iodfarbzahl, üblicherweise von maximal 40 und finden Verwendung zur Herstellung von kompakten oder geschäumten Polyisocyanat-polyadditionsprodukten, vorzugsweise flexiblen, halbharten oder harten Urethan- oder Urethan-und Isocyanuratgruppen enthaltenden Schaumstoffen, die eine deutlich hellere Farbe aufweisen.

### Beispiele

### Beispiel 1 und Vergleichsbeispiele I bis IV

Zu einer Reaktionsmischung, die bestand aus
90 Gew.-Teilen Monochlorbenzol als Lösungsmittel,
max. 1 Gew.-Teil Phosgen und
Roh-MDI, das seinerseits enthielt:
   54,7 Gew.-% 4,4'-MDI,
   2,4 Gew.-% 2,4'-MDI,
   max. 1 Gew.-% 2,2'-MDI und
      Homologe mit mehr als zwei Isocyanatgruppen sowie nicht identifizierte Nebenprodukte, wobei sich die Gew.-Teile zu 100 Gew.-Teilen und die Gew.-% zu 100 Gew.-% addierten,
fügte man bei einer Temperatur von 130 bis 140°C Wasser, ein Polyoxypropylen-glykol der Hydroxylzahl 250 oder eine erfindungsgemäße Mischung aus Wasser und dem genannten Polyoxypropylenglykol.

Die Reaktionsmischung wurde 5 Minuten lang bei 130 bis 140°C gehalten, anschließend in ungefähr 20 Minuten auf 175°C erwärmt und bei dieser Temperatur das restliche Phosgen und das Monochlorbenzol unter vermindertem Druck (ca. 60 mbar) abdestilliert.

Das erhaltene Roh-MDI wurde nunmehr bei 170 bis 180°C und unter einem verminderten Druck von kleiner als 10 mbar 30 Minuten lang entchloriert.

Bei einer Viskosität von 200 mPa·s bei 25°C, gemessen nach DIN 53 019, wurden die Iodfarbzahl (IFZ) der Gesamtchlorgehalt TC und der Isocyanatgehalt des Roh-MDI gemessen, wobei zur Bestimmung der Iodfarbzahl nach DIN 6162 das Roh-MDI mit Monochlorbenzol im Volumenverhältnis 1:5 verdünnt wurde.

Die eingesetzte Wasser- und Polyoxypropylen-glykolmenge und die ermittelte Iodfarbzahl, der Gesamtchlorgehalt TC und der Isocyanatgehalt des Roh-MDI sind in Tabelle 1 zusammengefaßt.

**Tabelle 1**

| Vergleichsbeispiel | Polyoxypropylenglykol [Gew.-%, bezogen auf Roh-MDI] | Wasser [Gew.-%, bezogen auf Roh-MDI] | Roh-MDI | | |
|---|---|---|---|---|---|
| | | | IFZ [1:5] | TC [ppm] | Isocyanatgehalt [Gew.-%] |
| I | - | - | 80 | 1600 | 31,5 |
| II | - | 0,02 | 70 | 2000 | 31,3 |
| III | - | 0,07 | 30 | 2100 | 31,3 |
| IV | 0,4 | - | 30 | 2300 | 31,1 |
| Beispiel 1 | 0,4 | 0,02 | 25 | 1900 | 31,5 |

### Beispiel 2 und Vergleichsbeispiel V

Zu einer Reaktionsmischung, die bestand aus
90 Gew.-Teilen Monochlorbenzol als Lösungsmittel,
max. 1 Gew.-Teil Phosgen und
Roh-MDI, das seinerseits enthielt:
   55 Gew.-% 4,4'-MDI,
   3,4 Gew.-% 2,4'-MDI,
   max. 1 Gew.-% 2,2'-MDI und
      Homologe mit mehr als zwei Isocyanatgruppen sowie nicht identifizierte Nebenprodukte, wobei sich die Gew.-Teile zu 100 Gew.-Teilen und die Gew.-% zu 100 Gew.-% addierten,
fügte man bei einer Temperatur von 138 bis 142°C, ein mit Glycerin gestartetes Polyoxypropylen-polyol der Hydroxylzahl 560 oder eine erfindungsgemäße Mischung aus Wasser und dem genannten Polyoxypropylen-polyol.

Die Reaktionsmischung wurde 5 Minuten lang bei 130 bis 140°C gehalten, anschließend in ungefähr 20 Minuten auf 175°C erwärmt und bei dieser Temperatur das restliche Phosgen und das Monochlorbenzol unter vermindertem Druck (ca. 60 mbar) abdestilliert.

Analog den Angaben von Beispiel 1 wurde das erhaltene Roh-MDI entchloriert und mit einer Viskosität von 200 mPa·s die Iodfarbzahl (IFZ), der Gesamtchlorgehalt und der Isocyanatgehalt, jeweils bezogen auf das Roh-MDI-Gewicht, gemessen.

Die eingesetzte Wasser- und Polyoxypropylen-polyolmenge und die ermittelten Analysenwerte sind in Tabelle 2 zusammengefaßt.

**Tabelle 2**

| Vergleichsbeispiel | Polyoxypropylenpolyol [Gew.-%, bezogen auf Roh-MDI] | Wasser [Gew.-%, bezogen auf Roh-MDI] | Roh-MDI | | |
|---|---|---|---|---|---|
| | | | IFZ [1:5] | TC [ppm] | Isocyanatgehalt [Gew.-%] |
| V | 0,4 | - | 70 | 3000 | 30,9 |
| Beispiel 2 | 0,25 | 0,02 | 35 | 2700 | 31,3 |

## Patentansprüche

1. Verfahren zur Herstellung von Mischungen aus Diphenylmethandiisocyanaten und Polyphenyl-polymethylen-polyisocyanaten mit einer verminderten Iodfarbzahl und einem reduzierten Chlorgehalt durch Umsetzung der entsprechenden Mischungen aus Diphenylmethan-diaminen und Polyphenyl-polymethylen-polyaminen mit Phosgen in Gegenwart mindestens eines inerten organischen Lösungsmittels bei erhöhter Temperatur, nach beendeter Phosgenierung Abtrennung des überschüssigen Phosgens und Lösungsmittels und thermische Behandlung des Reaktionsprodukts, dadurch gekennzeichnet, daß man der Reaktionsmischung nach beendeter Phosgenierung in Gegenwart oder Abwesenheit des Phosgens eine Mischung aus
i) mehr als 0,01 bis 0,3 Gew.-% Wasser und
ii) 0,05 bis 5 Gew.-% mindestens eines ein- oder mehrwertigen Polyoxyalkylenalkohols oder Gemisches davon,
jeweils bezogen auf das Gewicht der Mischung aus Diphenylmethan-diisocyanaten und Polyphenyl-polymethylen-polyisocyanaten in der Reaktionsmischung, einverleibt.

2. Verfahren nach Anspruch 1, dadurch gekennzeichnet, daß die wirksame Menge der Mischung besteht aus
i) mehr als 0,01 bis weniger als 0,1 Gew.-% Wasser und
ii) 0,05 bis 5 Gew.-% mindestens eines ein- oder mehrwertigen Polyoxyalkylenalkohols,
wobei die Gew.-% jeweils bezogen sind auf das Gewicht der Mischung aus Diphenylmethan-diisocyanaten und Polyphenyl-polymethylen-polyisocyanaten in der Reaktionsmischung.

3. Verfahren nach einem der Ansprüche 1 bis 2, dadurch gekennzeichnet, daß die ein- oder mehrwertigen Polyoxyalkylenalkohole (ii) eine Hydroxylzahl von 20 bis 1800 besitzen.

4. Verfahren nach einem der Ansprüche 1 bis 3, dadurch gekennzeichnet, daß die mehrwertigen Polyoxyalkylenalkohole (ii) eine Funktionalität von 2 bis 3 und eine Hydroxylzahl von 200 bis 800 besitzen.

5. Verfahren nach einem der Ansprüche 1 bis 4, dadurch gekennzeichnet, daß die ein- oder mehrwertigen Polyoxyalkylenalkohole (ii) aus Polyoxyethylen-, Polyoxypropylen-, Polyoxypropylen-polyoxyethylenalkoholen oder Mischungen davon bestehen.

6. Verfahren nach einem der Ansprüche 1 bis 5, dadurch gekennzeichnet, daß man zur Herstellung der Mischungen aus Diphenylmethan-diisocyanaten und Polyphenyl-polymethylen-polyisocyanaten als inerte organische Lösungsmittel Monochlorbenzol, Dichlorbenzole oder Mischungen davon verwendet und die eingesetzten ein- oder mehrwertigen Polyoxyalkylenalkohole (ii) in diesen Lösungsmitteln zumindest teilweise löslich sind.

7. Verfahren nach einem der Ansprüche 1 bis 6, dadurch gekennzeichnet, daß man der Wasser und mindestens ein- und/oder mehrwertige Polyoxyalkylenalkohole (ii) enthaltenden Mischung aus Diphenylmethan-diisocyanaten und Polyphenyl-polymethylen-polyisocyanaten nach der Abtrennung des Phosgens und des inerten organischen Lösungsmittels und vor der thermischen Behandlung des Reaktionsprodukts mindestens ein Antioxidans auf Phenolbasis in einer Menge von maximal 5 Gew.-% und/oder mindestens ein Arylphosphit in einer Menge von maximal 5 Gew.-%, jeweils bezogen auf das Gewicht der Mischung aus Diphenylmethan-diisocyanaten und Polyphenylpolymethylen-polyisocyanate, einverleibt.

8. Verfahren nach Anspruch 1, dadurch gekennzeichnet, daß man der Reaktionsmischung nach beendeter Phosgenierung und Abtrennung von überschüssigem Phosgen eine Mischung aus mehr als 0,01 bis weniger als 0,1 Gew.-% Wasser (i) und 0,05 bis 5 Gew.-% mindestens eines Polyoxyalkylenalkohols (ii) mit einer Funktionalität von 2 bis 3 und einer Hydroxylzahl von 200 bis 800, bezogen auf das Gewicht der Mischung aus Diphenylmethan-diisocyanaten und Polyphenyl-polymethylen-polyisocyanaten in der Reaktionsmischung, einverleibt, danach das noch vorhandene restliche Phosgen und das inerte organische Lösungsmittel abdestilliert, der Reaktionsmischung 0 bis 5 Gew.-% Di-tert.-butyl-p-kresol und/oder Triphenylphosphit, bezogen auf das Gewicht der Mischung aus Diphenylmethan-diisocyanaten und Polyphenyl-polymethylen-polyisocyanaten, hinzufügt und danach das Reaktionsprodukt thermisch behandelt.

## Claims

1. A process for preparing mixtures of diphenylmethane diisocyanates and polyphenylpolymethylene polyisocyanates having a reduced iodine color number and a reduced chlorine content by reacting the corresponding mixtures of diphenylmethanediamines and polyphenylpolymethylenepolyamines with phosgene in the presence of at least one inert organic solvent at elevated temperature, separating off the excess phosgene and solvent after phosgenation is complete and thermally treating the reaction product, wherein a mixture of
i) from more than 0.01 to 0.3 % by weight of water and
ii) from 0.05 to 5 % by weight of at least one monohydric or polyhydric polyoxyalkylene alcohol or mixture thereof,
in each case based on the weight of the mixture of diphenylmethane diisocyanates and polyphenylpolymethylene polyisocyanates in the reaction mixture, is incorporated into the reaction mixture after phosgenation is complete, in the presence or absence of the phosgene.

2. A process as claimed in claim 1, wherein the effective amount of the mixture comprises
i) from more than 0.01 to less than 0.1 % by weight of water and
ii) from 0.05 to 5 % by weight of at least one monohydric or polyhydric polyoxyalkylene alcohol,
where the percentages by weight are in each case based on the weight of the mixture of diphenylmethane diisocyanates and polyphenylpolymethylene polyisocyanates in the reaction mixture.

3. A process as claimed in claim 1 or 2, wherein the monohydric or polyhydric polyoxyalkylene alchols (ii) have a hydroxyl number of from 20 to 1800.

4. A process as claimed in any of claims 1 to 3, wherein the polyhydric polyoxyalkylene alcohols (ii) have a functionality of from 2 to 3 and a hydroxyl number of from 200 to 800.

5. A process as claimed in any of claims 1 to 4, wherein the monohydric or polyhydric polyoxyalkylene alcohols (ii) comprise polyoxyethylene, polyoxypropylene, polyoxypropylenepolyoxyethylene alcohols or mixtures thereof.

6. A process as claimed in any of claims 1 to 5, wherein the inert organic solvent used in preparing the mixtures of diphenylmethane diisocyanates and polyphenylpolymethylene polyisocyanates is monochlorobenzene, a dichlorobenzene or a mixture thereof and the monohydric or polyhydric polyoxyalkylene alcohols (ii) used are at least partially soluble in these solvents.

7. A process as claimed in any of claims 1 to 6, wherein the mixture of diphenylmethane diisocyanates and polyphenylpolymethylene polyisocyanates containing water and at least one monohydric and/or polyhydric polyoxyalkylene alcohol (ii) has incorporated into it, after the removal of the phosgene and the inert organic solvent and before the thermal treatment of the reaction product, at least one antioxidant based on phenol in an amount of at most 5 % by weight and/or at least one aryl phosphite in an amount of at most 5 % by weight, in each case based on the weight of the mixture of diphenylmethane diisocyanates and polyphenylpolymethylene polyisocyanates.

8. A process as claimed in claim 1, wherein the reaction mixture has incorporated into it, after phosgenation is complete and excess phosgene has been removed, a mixture of from more than 0.01 to less than 0.1 % by weight of water (i) and from 0.05 to 5 % by weight of at least one polyoxyalkylene alcohol (ii) having a functionality of from 2 to 3 and a hydroxyl number of from 200 to 800, based on the weight of the mixture of diphenylmethane diisocyanates and polyphenylpolymethylene polyisocyanates in the reaction mixture, the remaining phosgene still present and the inert organic solvent are then distilled off, the reaction mixture has added to it from 0 to 5 % by weight of di-tert-butyl-p-cresol and/or triphenyl phosphite, based on the weight of the mixture of diphenylmethane diisocyanates and polyphenylpolymethylene polyisocyanates, and the reaction product is then thermally treated.

## Revendications

1. Procédé pour la préparation de mélanges de diphénylméthane-diisocyanates et de polyphényl-polyméthylène-polyisocyanates possédant un indice d'iode inférieur et une teneur réduite en chlore, par mise en réaction des mélanges correspondants de diphénylméthanediamines et de polyphényl-polyméthylène-polyamines avec du phosgène en présence d'au moins un solvant organique inerte à une température élevée, par séparation du phosgène en excès et du solvant et par traitement thermique du produit réactionnel, au terme de la phosgénation, caractérisé en ce qu'on incorpore dans le mélange réactionnel, au terme de la phosgénation, en présence ou en l'absence du phosgène, un mélange constitué
i) à concurrence de plus de 0,01 à 0,3% en poids, par de l'eau et
ii) à concurrence de 0,05 à 5% en poids, par au moins un polyoxyalkylène-alcool monovalent ou polyvalent ou par un de ses mélanges,
chaque fois rapportés au poids du mélange de diphénylméthane-diisocyanates et de polyphényl-polyméthylène-polyisocyanates dans le mélange réactionnel.

2. Procédé selon la revendication 1, caractérisé en ce que la quantité efficace du mélange est constituée
i) à concurrence de plus de 0,01 à moins de 0,1% en poids, par de l'eau et
ii) à concurrence de 0,05 à 5% en poids, par au moins un polyoxyalkylène-alcool monovalent ou polyvalent,
les pour cent en poids étant chaque fois rapportés au poids du mélange de diphénylméthane-diisocyanates et de polyphényl-polyméthylène-polyisocyanates dans le mélange réactionnel.

3. Procédé selon l'une quelconque des revendications 1 à 2, caractérisé en ce que les polyoxyalkylène-alcools monovalents ou polyvalents (ii) possèdent un indice d'hydroxyle de 20 à 1.800.

4. Procédé selon l'une quelconque des revendications 1 à 3, caractérisé en ce que les polyoxyalkylène-alcools polyvalents (ii) possèdent une fonctionnalité de 2 à 3 et un indice d'hydroxyle de 200 à 800.

5. Procédé selon l'une quelconque des revendications 1 à 4, caractérisé en ce que les polyoxyalkylène-alcools monovalents ou polyvalents (ii) sont constitués par des polyoxyéthylène-alcools, par des polyoxypropylène-alcools, par des polyoxypropylène-polyoxyéthylène-alcools ou encore par leurs mélanges.

6. Procédé selon l'une quelconque des revendications 1 à 5, caractérisé en ce qu'on utilise, pour la préparation des mélanges de diphénylméthane-diisocyanates et de polyphényl-polyméthylène-polyisocyanates, à titre de solvants organiques inertes, du monochlorobenzène, des dichlorobenzènes ou encore des mélanges de ces derniers, les polyoxyalkylène-alcools monovalents ou polyvalents (ii) mis en oeuvre étant au moins partiellement solubles dans ces solvants.

7. Procédé selon l'une quelconque des revendications 1 à 6, caractérisé en ce qu'on incorpore, dans le mélange de diphénylméthane-diisocyanates et de polyphényl-polyméthylène-polyisocyanates, contenant de l'eau et au moins des polyoxyalkylène-alcools monovalents et/ou polyvalents (ii), après la séparation du phosgène et du solvant organique inerte et avant le traitement thermique du produit réactionnel, au moins un antioxydant à base de phénol en une quantité maximale de 5% en poids et/ou au moins un arylphosphite en une quantité maximale de 5% en poids, chaque fois rapportés au poids du mélange de diphénylméthane-diisocyanates et de polyphényl-polyméthylène-polyisocyanates.

8. Procédé selon la revendication 1, caractérisé en ce qu'on incorpore dans le mélange réactionnel, au terme de la phosgénation et après la séparation du phosgène en excès, un mélange constitué, à concurrence de plus de 0,01 à moins de 0,1% en poids, par de l'eau (i) et, à concurrence de 0,05 à 5% en poids, par au moins un polyoxyalkylène-alcool (ii) possédant une fonctionnalité de 2 à 3 et un indice d'hydroxyle de 200 à 800, rapportés au poids du mélange de diphénylméthane-diisocyanates et de polyphényl-polyméthylène-polyisocyanates dans le mélange réactionnel, on sépare ensuite par distillation le phosgène résiduel encore présent, ainsi que le solvant organique inerte, on ajoute au mélange réactionnel, à concurrence de 0 à 5% en poids, du di-tert-butyl-p-crésol et/ou du triphénylphosphite, rapportés au poids du mélange de diphénylméthane-diisocyanates et de polyphényl-polyméthylène-polyisocyanates, puis on soumet le produit réactionnel à un traitement thermique.
